(19) Europäisches Patentamt
European Patent Office
Office européen des brevets

(11) Numéro de publication: 0 335 758 A2

# (12) DEMANDE DE BREVET EUROPEEN

(21) Numéro de dépôt: 89400519.8

(22) Date de dépôt: 24.02.89

(51) Int. Cl.[4]: C 07 D 207/48
A 61 K 31/40

(30) Priorité: 26.02.88 IT 1956088

(43) Date de publication de la demande:
04.10.89 Bulletin 89/40

(84) Etats contractants désignés:
AT BE CH DE ES FR GB GR IT LI LU NL SE

(71) Demandeur: ROUSSEL-UCLAF
35, boulevard des Invalides
F-75007 Paris (FR)

(72) Inventeur: Galliani, Giulio
13, via Silva
Monza (MI) (IT)

Barzaghi, Fernando
Via Monteverdi, 21
I-20052 Monza Milan (IT)

Fortin, Michel
12, Passage Cottin
F-75018 Paris (FR)

Gorini, Carlo
Via Orcagna, 4
Milano (IT)

Toja, Emilio
Via Plezzo, 80
Milano (IT)

(74) Mandataire: Tonnellier, Marie-José et al
111, route de Noisy B.P. no 9
F-93230 Romainville (FR)

Revendications pour les Etats contractants suivants: ES + GR.

## (54) Nouveaux dérivés de la 1-arylsulfonyl 2-pyrrolidinone, leur procédé de préparation et les nouveaux intermédiaires ainsi obtenus, leur application comme médicaments et les compositions les renfermant.

(57) L'invention concerne les composés de formule (I) :

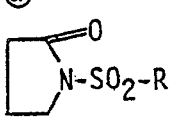

(I)

dans laquelle R représente

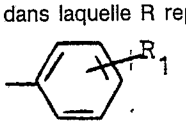

où $R_1$ est soit alcoyle ($C_{1-8}$), soit

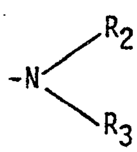

où $R_2$ et $R_3$ sont hydrogène, alcoyle ($C_{1-8}$) ou ensemble avec N représentent un hétérocyclique contenant éventuellement un autre hétéroatome, soit $NO_2$, OR′ où R′ est hydrogène, alcoyle ($C_{1-8}$) ou aryle, soit $SR_4$, $S(O)R_5$ où $R_4$ et $R_5$ sont alcoyle ($C_{1-8}$), ou bien R représente un naphtyle éventuellement substitué par $R'_1$, $R'_1$ ayant les valeurs indiquées pour $R_1$, leur procédé de préparation, leur application comme médicaments utiles dans le traitement des troubles spasmodiques divers et les compositions pharmaceutiques les renfermant.

EP 0 335 758 A2

## Description

**Nouveaux dérivés de la 1-arylsulfonyl 2-pyrrolidinone, leur procédé de préparation et les nouveaux intermédiaires ainsi obtenus, leur application comme médicaments et les compositions les renfermant.**

La présente invention concerne de nouveaux dérivés de la 1-arylsulfonyl 2-pyrrolidinone, leur procédé de préparation et les nouveaux intermédiaires ainsi obtenus, leur application comme médicaments et les compositions les renfermant.

L'invention a pour objet les composés de formule (I) :

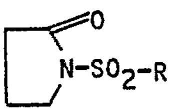

(I)

dans laquelle R représente

- ou bien un radical

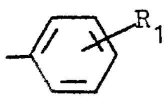

dans laquel $R_1$ en position quelconque sur le noyau phényle représente
soit un radical alcoyle renfermant jusqu'à 8 atomes de carbone linéaire, ramifié ou cyclique, saturé ou insaturé,
soit un radical

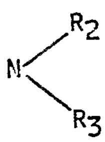

dans lequel $R_2$ et $R_3$ identiques ou différents représentent un atome d'hydrogène, un radical alcoyle linéaire, saturé ou insaturé renfermant jusqu'à 8 atomes de carbone, ou forment ensemble avec l'atome d'azote auquel ils sont liés un radical hétérocyclique renfermant éventuellement un autre hétéroatome,
soit un radical $NO_2$,
soit un radical OR', R' représentant un atome d'hydrogène, un radical alcoyle linéaire ramifié ou cyclique renfermant jusqu'à 8 atomes de carbone, ou un radical aryle renfermant jusqu'à 14 atomes de carbone,
soit un radical $SR_4$ ou $S(O)R_5$, $R_4$ et $R_5$ représentant un radical alcoyle linéaire, ramifié ou cyclique, saturé ou insaturé renfermant jusqu'à 8 atomes de carbone,

- ou bien R représente un radical naphtyle, éventuellement substitué par un radical $R'_1$, $R'_1$ pouvant prendre l'une des valeurs indiquées ci-dessus pour $R_1$.

Par radical alcoyle, on entend de préférence un radical alcoyle renfermant de 1 à 5 atomes de carbone, par exemple le radical méthyle, éthyle, n-propyle, isopropyle, n-butyle, isobutyle, tert-butyle, n-pentyle, n-hexyle, cyclopro-pyle, cyclobutyle, cyclopentyle ou cyclohexyle.

Par radical alcoyle insaturé, on entend de préférence un radical éthényle, propényle et butényle. Lorsque $R_2$ et $R_3$ forment avec l'atome d'azote auquel ils sont liés un radical hétérocyclique renfermant éventuellement un autre hétéroatome, il s'agit de préférence d'un radical piperidyle, piperazinyle, morpholinyle ou pyrrolidinyle.

Parmi les composés préférés de l'invention, on peut citer les composés de formule (I) dans lesquels R représente un radical

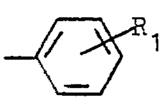

$R_1$ conservant la même signification, et notamment ceux dans lesquels le radical $R_1$ est en position 4. On peut également citer les composés de formule (I) dans lesquels $R_1$ représente un radical alcoyle linéaire ou ramifié renfermant jusqu'à 8 atomes de carbone et notamment un radical tert-butyle ou encore ceux dans lesquels $R_1$ représente un radical

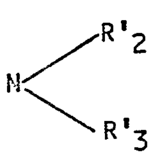

dans lequel $R'_2$ et $R'_3$ représentent un radical alcoyle linéaire ou ramifié renfermant jusqu'à 8 atomes de carbone ou forment avec l'atome d'azote auquel ils sont liés un radical hétérocyclique, ou encore ceux dans lesquels $R_1$ représente un radical $SR_4$, $R_4$ représentant un radical alcoyle linéaire ou ramifié renfermant jusqu'à 4 atomes de carbone et notamment un radical méthyle.

Parmi les composés préférés de l'invention, on peut citer les composés dont la préparation est donnée ci-après dans la partie expérimentale, et tout spécialement les composés des exemples 1, 2, 3, 4 et 10.

Les composés de formule (I) présentent d'intéressantes propriétés pharmacologiques et notamment une activité antimuscarinique spécifique et sélective.

L'invention a donc pour objet les produits de formule (I) en tant que médicaments, utiles notamment pour le traitement des troubles spasmodiques divers en gastro-entérologie, en gynécologie, en obstétrique, en urologie, en hépatologie et en radiologie.

L'invention a plus particulièrement pour objet en tant que médicament, les produits des exemples 1, 2, 3, 4 et 10.

La posologie usuelle est variable selon l'affection en cause, le sujet traité et la voie d'administration, elle peut être comprise entre 10 mg et 1 g par jour, par exemple entre 30 et 60 mg par jour en une ou plusieurs prises pour le produit de l'exemple 1 administré par voie orale.

La présente invention a également pour objet les compositions pharmaceutiques renfermant comme principe actif au moins un produit de formule (I). Les compositions pharmaceutiques de l'invention peuvent être solides ou liquides et se présenter sous les formes pharmaceutiques couramment utilisées en médecine humaine, comme par exemple, les comprimés simples ou dragéifiés, les gélules, les granulés, les suppositoires, les préparations injectables ; elles sont préparées selon les méthodes usuelles.

Le ou les principes actifs peuvent y être incorporés à des excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifiants, les conservateurs.

L'invention a également pour objet un procédé de préparation des composés de formule (I) caractérisé en ce que l'on soumet la 2-pyrrolidinone

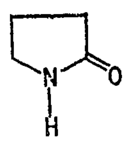

à l'action d'un composé de formule (II) :

$R\text{-}SO_2Hal$ (II)

dans laquelle Hal représente un atome de chlore ou de brome, et R conserve la même signification que précédemment, pour obtenir le composé de formule (I) correspondant.

Dans un mode de réalisation préférée du procédé de l'invention, la réaction entre la 2-pyrrolidinone et le produit de formule (II) est effectuée :

a) en présence d'une base forte comme le butyllithium, un hydrure alcalin, comme l'hydrure de sodium ou le bis(triméthylsilyl) amidure de sodium ;

b) au sein d'un solvant choisi dans le group constitué par le tétrahydrofuranne, le benzène, le diméthylformamide, le diméthylsulfoxyde, l'éther monoéthylique du diéthylène glycol, ou l'éther diéthylique du diéthylène glycol.

L'invention a également pour objet un procédé de préparation des composés de formule (I), caractérisé en ce que l'on somet l'acide 4-amino butyrique à l'action d'un composé de formule (II) :

$R\text{-}SO_2\text{-}Hal$ (II)

dans laquelle R et Hal ont la signification indiquée précédemment pour obtenir un composé de formule (III') :

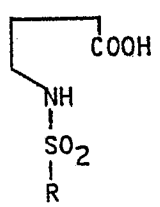

(III')

dans laquelle R a la signification indiquée précédemment, que l'on cyclise pour obtenir le produit de formule (I) correspondant.

Dans un mode de réalisation préférée du procédé ci-dessus décrit :

- la réaction de l'acide 4-amino butyrique avec le composé de formule (II) est effectuée en présence d'une base minérale telle que la soude ou la potasse au sein d'un solvant organique tel que le tétrahydrofuranne ;
- la cyclisation du composé de formule (III) est réalisée en présence d'un agent déshydratant tel que l'anhydride acétique, l'anhydride phosphorique, l'acide phosphorique ou encore l'hexaméthyldisilasane.

L'invention a également pour objet à titre de produits industriels nouveaux, les produits de formule (III) tels

que définis précédemment.

Les exemples suivants illustrent l'invention sans toutefois la limiter.

**Exemple 1 : 1-(4-terbutyl benzènesulfonyl) 2-pyrrolidinone.**

On refroidit à -5¤C, 1,65 g de 2-pyrrolidinone en solution dans 75 cm3 de tétrahydrofuranne et ajoute 12,1 cm3 d'une solution 1,6M de n-butyllithium dans l'hexane en maintenant la température entre -5¤C et 0¤C. On agite 25 minutes à -5¤C; refroidit à -20¤C et ajoute 4,5 g de chlorure de 4-terbutyl phénylsulfonyl (Recueil Trav. Chim. Pays-Bas, 53, 1101 (1934)). On laisse refroidir à température ambiante, évapore le tétrahydrofuranne sous pression réduite, reprend le résidu à l'eau, filtre, cristallise dans l'éthanol. On obtient 2,75 g de produit attendu. F = 131-133¤C.

Analyse : $C_{14}H_{19}NO_3S$ : 281,376

| | | | | |
|---|---|---|---|---|
| Calculé | : | C% 59,76 | H% 6,81 | N% 4,98 |
| Trouvé | : | 59,62 | 6,78 | 4,79 |

**Exemple 2 : 1-[4-(diéthylamino) benzènesulfonyl] 2-pyrrolidinone.**

On refroidit à -10¤C 0,69 g de 2-pyrrolidinone en solution dans 25 cm3 de tétrahydrofuranne, ajoute 4,89 cm3 d'une solution 1,6M de N-butyllithium dans le n-hexane en opérant à une température inférieure à 5¤C. On agite 20 minutes, refroidit à -25¤C, verse goutte à goutte une solution de 2 g de chlorure de diéthylaminobenzènesulfonyle dans 15 cm3 de tétrahydrofuranne en maintenant la température inférieure à -20¤C. On laisse revenir à température ordinaire et agite pendant 2 heures. On élimine le solvant sous pression réduite, chromatographie le résidu sur silice (éluant : acétate d'éthyle-n-hexane 1-1), reprend le résidu à l'éther isopropylique et obtient 0,74 g de produit attendu. F = 128¤130¤C.

Analyse : $C_{14}H_{20}N_2O_3S$ : 296,392

| | | | | |
|---|---|---|---|---|
| Calculé | : | C% 56,73 | H% 6,8 | N% 9,45 |
| Trouvé | : | 56,59 | 6,73 | 9,38 |

Le chlorure de 4-(diéthylamino) benzènesulfonyle utilisé au départ de l'exemple a été préparé comme suit :

On mélange 24,84 g de l'acide 4-diéthylaminobenzènesulfonique (Liebigs Ann. Chem. (1982) 282) et 22,56 g de pentachlorure de phosphore dans 350 cm3 de chlorure de méthylène et chauffe au reflux pendant 4 heures. On refroidit à température ambiante, évapore le solvant sous pression réduite, reprend au toluène, filtre sur célite et concentre à sec sous pression réduite. On obtient 20,39 g de produit attendu.

**Exemple 3 : 1-[4-(diméthylamino) benzènesulfonyl] 2-pyrrolidinone.**

A une solution comprenant 6,11 g de 2-pyrrolidinone et 200 cm3 de dioxane, on ajoute 3,44 g d'hydrure de sodium (à 55-60% dans l'huile) et agite 1 heure à température ambiante. On ajoute goutte à goutte 15,76 cm3 de chlorure de 4-diméthylaminobenzènesulfonyle [Beritche 43, 3038 (1910)] en solution dans 250 cm3 de dioxane et agite 1 heure à température ambiante. On filtre le chlorure de sodium sur célite, évapore le dioxane sous pression réduite, cristallise le résidu dans l'acétone et obtient 3,90 g de produit attendu. F = 202¤-204¤C.

Analyse : $C_{12}H_{16}N_2O_3S$ : 268,340

| | | | | |
|---|---|---|---|---|
| Calculé | : | C% 53,71 | H% 6,01 | N% 10,44 |
| Trouvé | : | 53,92 | 5,97 | 10,50 |

(Soluble dans le chloroforme ; peu soluble dans l'acétone, le benzène et l'alcool à 95¤ ; insoluble dans l'éther éthylique, l'eau, l'acide chlorhydrique 2N, la soude 2N).

**Exemple 4 : 1[4-(méthylthio) benzènesulfonyl] 2-pyrrolidinone.**

A 2,29 g de 2-pyrrolidinone en solution dans 160 cm3 de tétrahydrofuranne anhydre refroidie à -30¤C, on ajoute goutte à goutte 16,95 cm3 d'une solution 1,6M de n-butyllithium dans le n-hexane en maintenant à -30¤C. On agite 30 minutes, verse goutte à goutte une solution comprenant 6 g de chlorure de 4-méthylthiobenzènesulfonyle [J. Chem. Soc. (1948) 604] dans 10 cm3 de tétrahydrofuranne en opérant entre -30¤C et -25¤C. On laisse revenir à température ambiante, évapore le solvant sous pression réduite, reprend le résidu à l'eau et filtre le solide. Après cristallisation dans l'isopropanol, on obtient 3,80 g de produit attendu. F = 123¤-125¤C.

Analyse : $C_{11}H_{13}NO_3S_2$ : 271,36

| | | | | |
|---|---|---|---|---|
| Calculé | : | C% 48,69 | H% 4,83 | N% 5,16 |
| Trouvé | : | 48,91 | 4,69 | 5,22 |

**Exemple 5 : 1-(4-isopropyloxybenzènesulfonyl) 2-pyrrolidinone.**

A 1,3 g de 2-pyrrolidinone en solution dans 90 cm3 de tétrahydrofuranne puis redroidie à -30¤C, on ajoute 9,6 cm3 d'une solution 1,6M de n-butyllithium dans l'hexane en maintenant la température entre -20¤C et -30¤C. On agite 1 heure à -30¤C, verse goutte à goutte à cette température 4 g de chlorure de 4-isopropyloxybenzènesulfonyle [Helv. Chim. Acta 39, 1579 (1956)] en solution dans 6 cm3 de tétrahydrofuranne. On agite pendant 1 heure à -30¤C, laisse revenir à température ambiante, évapore le solvant sous pression réduite et chromatographie le résidu sur silice (éluant : cyclohexane-acétate d'éthyle 7-3). Après cristallisation dans l'isopropanol, on obtient 1,5 g de produit attendu. F = 88¤-90¤C.

Analyse : $C_{13}H_{17}NO_4S$ : 283,35

| | | | | |
|---|---|---|---|---|
| Calculé | : | C% 55,10 | H% 6,05 | N% 4,94 |
| Trouvé | : | 55,07 | 5,98 | 4,90 |

**Exemple 6 : 1-[4-méthylsulfinyl) benzènesulfonyl] 2-pyrrolidinone.**

A 3,4 g de 1-(4-méthylthiobenzènesulfonyl) pyrrolidin-2-one préparé comme à l'exemple 4 en solution dans 34 cm3 de chlorure de méthylène, on ajoute une solution comprenant 2,41 g d'acide m-chloroperbenzoïque dans 48 cm3 de chlorure de méthylène à une température ne dépassant pas 25¤C. On agite à température ambiante pendant 30 minutes, traite ensuite le milieu réactionnel avec une solution aqueuse de sulfite de sodium à 10%. On sépare la phase organique, le lave avec une solution aqueuse de bicarbonate de soude à 5%, puis à l'eau. On sèche, évapore le solvant sous pression réduite, cristallise le résidu dans l'éthanol 95 et obtient 1,70 g de produit attendu. F = 127¤129¤C.

Analyse : $C_{11}H_{13}NO_4S_2$ : 287,36

| | | | | |
|---|---|---|---|---|
| Calculé | : | C% 45,98 | H% 4,56 | N% 4,87 |
| Trouvé | : | 45,87 | 4,60 | 4,81 |

**Exemple 7 : 1-(3-méthoxybenzènesulfonyl) 2-pyrrolidinone.**

A 2,22g de 2-pyrrolidinone en solution dans 80 cm3 de tétrahydrofuranne et refroidie à -25¤C, on ajoute 15,76 cm3 d'un solution 1,6M de N-butyllithium dans l'hexane en maintenant la température entre -25¤C et -20¤C. On agite 30 minutes à -25¤C, verse goutte à goutte une solution de 5,40 g de chlorure de 3-méthoxybenzènesulfonyle [J. Chem. Soc. P.T.2., 579 (1982)] dans 40 cm3 de tétrahydrofuranne en opérant entre -25¤C et -20¤C. On agite 30 minutes à -25¤C, laisse revenir à température am biante, évapore le solvant sous pression réduite, reprend le résidu dans l'eau, filtre et cristallise dans l'isopropanol. On obtient 3,5 g de produit attendu. F = 108¤-109¤C.

Analyse : $C_{11}H_{13}NO_4S$ : 255,298

| | | | | |
|---|---|---|---|---|
| Calculé | : | C% 51,75 | H% 5,13 | N% 5,49 |
| Trouvé | : | 51,84 | 5,12 | 5,54 |

**Exemple 8 : 1-(2-naphtylsulfonyl) 2-pyrrolidinone.**

A 2,13 g de 2-pyrrolidinone en solution dans 80 cm3 de tétrahydrofuranne et refroidie à -10¤C, on ajoute 15,6 cm3 d'une solution 1,6M de butyllithium dans l'hexane en maintenant la température entre -5¤C et +5¤C. On agite à -5¤C pendant 25 minutes, puis ajoute 6,12 g de chlorure de béta-naphtalènesulfonyle sans dépasser 0¤C. On laisse ensuite revenir à tempértature ambiante, évapore le solvant sous pression réduite, reprend le résidu à l'acétate d'éthyle, filtre le chlorure de lithium et évapore sous pression réduite. On reprend à l'eau et filtre. Enfin on cristallise dans l'isopropanol et obtient 4 g de produit attendu. F = 118¤-120¤C.

Analyse : $C_{14}H_{13}NO_3S$ : 275,3

| | | | | |
|---|---|---|---|---|
| Calculé | : | C% 61,07 | H% 4,76 | N% 5,09 |
| Trouvé | : | 60,91 | 4,72 | 4,99 |

**Exemple 9 : 1-[(4-pyrrolidinyl) phénylsulfonyl] 2-pyrrolidinone.**

**Stade A :** Acide 4-(4-pyrrolidinylphénylsulfonylamino) butyrique.

A une solution comprenant 2,06 g d'acide 4-aminobutyrique et 2,4 g de soude dans 40 cm3 d'eau, on ajoute 4,9 g de chlorure de 4-pyrrolidinebenzènesulfonyle (Chem. Abst. 46, 8647 F) puis 50 cm3 de tétrahydrofuranne afin d'obtenir une solution parfaite. On agite 24 heures à température anbiante, acidifie avec de l'acide acétique et évapore le solvant à température ambiante. On extrait au chloroforme, sèche la phase organique sur sulfate de soude, filtre et évapore à sec. On obtient 2,4 g de produit attendu. F = 161¤163¤C.

Analyse : $C_{14}H_{20}N_2O_4S$ : 312,39

| | | | | |
|---|---|---|---|---|
| Calculé | : | C% 53,83 | H% 6,45 | N% 8,97 |
| Trouvé | : | 53,16 | 6,25 | 8,72 |

**Stade B :** 1-[(4-pyrrolidinyl) phénylsulfonyl] 2-pyrrolidinone.

On chauffe 4 heures au reflux 2,5 g de produit préparé comme au stade A et 2,5 g d'acétate de sodium dans 50 cm3 d'anhydride acétique. On refroidit et évapore à sec. On reprend le résidu dans 150 cm3 de benzène, chauffe à ébullition, filtre sur charbon actif. Par addition de n-hexane, on obtient un précipité que l'on filtre et lave à l'hexane. On obtient 1,7 g de produit attendu. F = 235¤-237¤C.

Analyse : $C_{14}H_{18}N_2O_3S$ : 294,38

| | | | | |
|---|---|---|---|---|
| Calculé | : | C% 57,12 | H% 6,16 | N% 9,51 |
| Trouvé | : | 56,77 | 6,24 | 9,31 |

**Exemple 10 : 1-[4-(1-pipéridinyl) phénylsulfonyl] 2-pyrrolidinone.**

**Stade A :** Acide 4-(4-pipéridinephénylsulfonylamino) butyrique.

On ajoute 1 g de chlorure de 4-pipéridinebenzènesulfonyle à une solution comprenant 0,39 de d'acide 4-aminobutyrique 0,462 g de soude en solution dans 10 cm3 d'eau puis 5 cm3 de tétrahydrofuranne de façon à obtenir une solution. On agite 24 heures à température ambiante, acidifie à l'aide d'acide acétique, évapore le solvant, extrait au chloroforme, sèche la phase organique sur sulfate de sodium, filtre et évapore à sec. Après cristallisation dans l'acétate d'éthyle, on obtient 0,5 g de produit attendu. F = 130¤-132¤C.

Analyse : $C_{15}H_{22}N_2O_4S$ : 326,43

| | | | | |
|---|---|---|---|---|
| Calculé | : | C% 55,19 | H% 6,79 | N% 8,58 |
| Trouvé | : | 54,69 | 6,72 | 8,37 |

**Stade B :** 1-[4-(1-pipéridinyl) phénylsulfonyl] 2-pyrrolidinone.

On porte 3 heures au reflux un mélange constitué de 3 g de produit obtenu comme au stade A et 3 g d'acétate de sodium dans 60 cm3 d'anhydride acétique. On refroidit à température ambiante et évapore à sec. On extrait au chloroforme et à l'eau, sépare la phase organique, la sèche sur sulfate de sodium, filtre et évapore. On obtient 2,5 g de produit attendu. F = 168¤-170¤C. Après cristallisation dans l'isopropanol, on obtient 1,80 g de produit. F = 170¤-172¤C.

Analyse : $C_{15}H_{20}N_2O_3S$ : 308,41

| | | | | |
|---|---|---|---|---|
| Calculé | : | C% 58,42 | H% 6,54 | N% 9,08 |
| Trouvé | : | 58,31 | 6,46 | 8,89 |

Le chlorure de 4-pipéridinebenzènesulfonyle utilisé au départ de l'exemple 10 a été préparé comme suit.

On ajoute 9,3 g de dioxane dans une solution comprenant 8,46 g d'anhydride sulfurique dans 45 cm3 de chlorure de méthylène refroidie à 0¤C/+5¤C puis ajoute à la même température 17,1 g de N-phénylpipéridine en solution dans 45 cm3 de chlorure de méthylène. On laisse revenir à température ambiante puis chauffe au reflux pendant 1 heure. On évapore à sec, reprend le résidu par une solution de carbonate de sodium à 10%, lave au benzène et concentre la phase aqueuse, sèche le résidu, traite par 200 cm3 d'oxychlorure de phosphore et 21,8 g de pentachlorure de phosphore pendant 12 heures à température ambiante. On évapore à sec, reprend le résidu avec du chloroforme et de l'eau, sépare la phase organique, sèche sur sulfate de sodium, filtre et évapore à sec. On obtient 19 g de produit que l'on utilise tel quel.

**Exemple 11 : 1-[(4-morpholino) phénylsulfonyl] 2-pyrrolidinone.**

**Stade A** : Acide (4-morpholinophénylsulfonylamino) butyrique.

A une solution comprenant 3,94 g d'acide 4-amino butyrique et 4,6 g de soude dans 80 cm3 d'eau, on ajoute 10 g de chlorure 4-morpholino benzène sulfonyle puis 70 cm3 de tétrahydrofuranne afin d'obtenir une solution. La température s'élève de 20 à 27¤C. On laisse revenir à température ambiante et maintient sous agitation pendant 24 heures. On acidifie à l'aide d'acide acétique, évapore le solvant sous pression réduite, reprend le résidu avec 50 cm3 d'eau distillée, filtre le précipité, le lave à l'eau, le sèche et obtient 7,6 g de produit attendu, F = 163¤-165¤C, que l'on recristallise dans l'acétate d'éthyle. F = 167¤-168¤C.

Analyse : $C_{14}H_{20}N_2O_5S$ : 328,39

| | | | | |
|---|---|---|---|---|
| Calculé | : | C% 51,20 | H% 6,14 | N% 8,53 |
| Trouvé | : | 51,21 | 6,07 | 8,61 |

**Stade B** : 1-[(4-morpholino) phénylsulfonyl] 2-pyrrolidinone.

On chauffe 4 heures au reflux 6,2 g de produit obtenu au stade A, 6,2 g d'acétate de sodium et 124 cm3 d'anhydride acétique. On refroidit à température ambiante, évapore à sec, reprend le résidu avec 50 cm3 d'eau, filtre, lave avec 10 cm3 d'eau et sèche. On obtient 5,65 g de produit attendu. F = 208¤-210¤C. Après recristallisation dans l'acétone, on obtient 4,3 g de produit fondant à 210¤211¤C.

Analyse : $C_{14}H_{18}N_2O_4S$ : 310,38

| | | | | |
|---|---|---|---|---|
| Calculé | : | C% 54,17 | H% 5,85 | N% 9,03 |
| Trouvé | : | 54,30 | 5,91 | 8,92 |

Le chlorure de 4-morpholinobenzènesulfonyle utilisé au départ de l'exemple 11 a été préparé comme suit.

A une solution comprenant 8,8 g d'anhydride sulfurique dans 100 cm3 de chlorure de méthylène et refroidie entre -5¤C et +3¤C, on ajoute goutte à goutte 9,68 g de dioxane puis 17,95 g de N-phénylmorpholine dissous dans 30 cm3 de chlorure de méthylène, sans que la température dépasse +5¤C. On laisse revenir à température ambiante, puis chauffe au reflux pendant 1 heure et demie. On refroidit à température ambiante, extrait à l'eau, neutralise la phase aqueuse avec du carbonate de sodium, évapore à sec et sèche le résidu sous pression réduite à 90¤C pendant 4 heures. On obtient 19,5 g de sel sodique que l'on traite par 21 g de pentachlorure de phosphore dans 100 cm3 de chlorure de méthylène en chauffant 4 heures au reflux. On refroidit à température ambiante, évapore à sec, reprend le résidu au benzène avec un peu d'eau, sépare la phase organique, la sèche sur sulfate de sodium, filtre et évapore le solvant. On obtient 11,5 g de produit attendu. F = 105¤-108¤C. Après cristallisation dans le benzène, on obtient le produit fondant à 120¤-122¤C.

Analyse : $C_{10}H_{12}ClNO_3S$ : 261,77

| | | | | |
|---|---|---|---|---|
| Calculé | : | C% 45,88 | H% 4,62 | N% 5,35 |
| Trouvé | : | 45,67 | 4,59 | 5,44 |

**Exemple 12 : 1-[(4-cyclopentyl) phénylsulfonyl] 2-pyrrolidinone.**

A une solution comprenant 1,02g soit 0,9 cm3 de 2-pyrrolidinone dans 50 cm3 de tétrahydrofuranne, refroidie à -70¤C, on verse 8 cm3 d'une solution 1,5M de n-butyllithium dans le n-hexane sans que la température dépasse -60¤C. Au bout de 15 minutes, on ajoute 3 g de chlorure de benzènesulfonyl 4-cyclopentyle en solution dans 12 cm3 de tétrahydrofuranne en maintenant la température entre -65¤ et -70¤C. On laisse revenir à température ambiante en 2 heures, évapore à sec, chromatographie le résidu sur silice (éluant : acétate d'éthyle-n-hexane 1-3) et obtient 2,6 g de produit attendu, F = 105¤-106¤C, qui après cristallisation dans l'isopropanol donne 2 g de produit fondant à 105¤-106¤C.

Analyse : $C_{15}H_{19}NO_3S$ : 293,40

| | | | | |
|---|---|---|---|---|
| Calculé | : | C% 61,41 | H% 6,53 | N% 4,77 |
| Trouvé | : | 61,22 | 6,71 | 5,06 |

Le chlorure de benzènesulfonyl 4-cyclopentyle utilisé au départ de l'exemple 12 a été préparé comme suit.

A 11,5 de phénylcyclopentane [Chem. Abstr. 51, 7317c (1957)], on ajoute entre +5¤C et +10¤C, 14,7 g soit 12 cm3 de chlorosulfonate de triméthylsilyle. On laisse revenir à température ambiante et agite pendant 2 heures. On évapore à sec, dissout le résidu dans 100 cm3 de chloroforme, traite avec 7,5 g de pentachlorure de phosphore et chauffe 2 heures au reflux. On évapore à sec, chromatographie le résidu huileux sur silice

(éluant : acétate d'éthyle-n-hexane 1-3) et obtient 3,3 g de produit attendu. F = 48¤-50¤C.

**Exemple 13 : 1-[(4-cyclohexyl) phénylsulfonyl] 2-pyrrolidinone.**

On opère comme à l'exemple 12, en utilisant 1,97 g de 2-pyrrolidinone dans 100 cm3 de tétrahydrofuranne, 15,5 cm3 d'une solution 1,5M de n-butyllithium dans l'hexane, 6 g de chlorure (4-cyclohexyl) benzènesulfonyl [J. Am. Chem. Soc. 62, 513 (1940)] dans 24 cm3 de tétrahydrofuranne. On obtient 3 g de produit attendu. F = 91¤-92¤C. Après cristallisation dans l'isopropanol, on obtient 2 g de produit fondant. F = 91¤-92¤C.

Analyse : $C_{16}H_{21}NO_3S$ : 307,42

| | | | | |
|---|---|---|---|---|
| Calculé | : | C% 62,51 | H% 6,88 | N% 4,56 |
| Trouvé | : | 62,29 | 6,74 | 4,69 |

**Exemple 14 : 1-[(4-dipropylamine) phénylsulfonyl] 2-pyrrolidinone.**

On opère comme à l'exemple 12, en utilisant 1,39 g de 2-pyrrolidinone dans 42 cm3 de tétrahydrofuranne, 10,8 cm3 d'une solution 1,5M de n-butyllithium dans le n-hexane, puis entre -20¤C et -10¤C 4,5 g de chlorure de (4-dipropylamino) benzènesulfonyle dans 25 cm3 de tétrahydrofuranne. On obtient 2 g de produit attendu. F = 88¤-90¤C. Après cristallisation dans l'isopropanol, on obtient 1,5 g de produit fondant à 92¤-93¤C.

Analyse : $C_{16}H_{24}N_2O_3S$ : 324,454

| | | | | |
|---|---|---|---|---|
| Calculé | : | C% 59,23 | H% 7,46 | N% 8,63 |
| Trouvé | : | 59,13 | 7,53 | 8,44 |

Le chlorure de (4-dipropylamino) benzènesulfonyle utilisé au départ de l'exemple 14 a été préparé comme suit.

On ajoute 10,2 g de (N,N-dipropylaniline) (Annalen der Chemie 214, 168) à une solution refroidie à +5¤C/+10¤C comprenant 10,86 g (soit 8,86 cm3) de chlorosulfonate de triméthylsilyl et 50 cm3 de chlorure de méthylène. On laisse revenir à température ambiante, évapore à sec, reprend le résidu à l'acétone, filtre, sèche et obtient 4,9 g d'acide auquel on ajoute 100 cm3 de chlorure de méthylène et 2,63 g de pentachlorure de phosphore et chauffe au reflux pendant 4 heures. On refroidit à température ambiante, évapore à sec, reprend le résidu huileux avec du benzène et de l'eau, sépare la phase organique, sèche sur sulfate de sodium, filtre et évapore à sec. On obtient 4,5 de produit attendu que l'on utilise tel quel.

**Exemple 15 : 1-[(4-dibutylamino) phénylsulfonyl] 2-pyrrolidinone.**

On opère comme à l'exemple 12 en utilisant 1,76 g de 2-pyrrolidinone dans 51 cm3 de tétrahydrofuranne, 13,8 cm3 d'une solution 1,5M de n-butyllithium dans le n-hexane à une température comprise entre -20¤C et -15¤C, puis 6,3 g de chlorure de 4-dibutylaminobenzènesulfonyle dans 35 cm3 de tétrahydrofuranne. Après chromatographie sur silice (éluant : acétate d'éthyle-n-hexane 1-1) et cristallisation dans l'isopropanol, on obtient 3 g de produit attendu. F = 73¤-74¤C.

Analyse : $C_{18}H_{28}N_2O_3S$ : 352,51

| | | | | |
|---|---|---|---|---|
| Calculé | : | C% 61,33 | H% 8,01 | N% 7,95 |
| Trouvé | : | 61,14 | 8,03 | 7,86 |

Le chlorure de 4-dibutylamino benzènesulfonyle utilisé au départ de l'exemple 15 a été préparé comme suit.

On opère comme indiqué pour la préparation du produit de départ de l'exemple 14 en utilisant 10 g de (N,N-dibutylaniline) (J. Chem. Soc. (1956), 3293), 9,19 g (soit 7,5 cm3 de chlorosulfonate de triméthylsilyle dans 50 cm3 de chlorure de méthylène. Après évaporation à sec, le résidu est repris dans 100 cm3 de chlorure de méthylène puis on ajoute 10,1 g de pentachlorure de phosphore, chauffe au reflux pendant 1 heure et demie, refroidit, évapore à sec, reprend le résidu dans l'eau et extrait au chloroforme. On sèche sur sulfate de sodium, filtre, évapore à sec, chromatographie le résidu sur silice (éluant : acétate d'éthyle-n-hexane 1-1) et obtient 6,3 g de produit attendu que l'on utilise tel quel.

**Exemple 16 : 1[(4-diisopropylamino) phénylsulfonyl] 2-pyrrolidinone.**

On opère comme à l'exemple 12 en utilisant 2,76 g de 2-pyrrolidinone dans 81 cm3 de tétrahydrofuranne, 21,7 cm3 d'une solution 1,5M de n-butyllithium dans le n-hexane à une température comprise entre -20¤C et -15¤C puis 9 g de chlorure de (4-diisopropylamino) benzènesulfonyle dans 45 cm3 de tétrahydrofuranne. Après chromatographie (éluant : acétate d'éthyle-n-hexane 1-2), on obtient 3,4 g de produit attendu. F = 140¤-145¤C puis 142¤-145¤C après cristallisation dans l'isopropanol.

Analyse : $C_{16}H_{24}N_2O_3$ : 324,45

| | | | | |
|---|---|---|---|---|
| Calculé | : | C% 59,23 | H% 7,46 | N% 8,63 |
| Trouvé | : | 59,09 | 7,38 | 8,57 |

Le chlorure de 4-diisopropylaminobenzènesulfonyle utilisé au départ de l'exemple 16 a été préparé comme suit.

On opère comme indiqué pour la préparation du produit de départ de l'exemple 14 en utilisant 13 g de (N,N-diisopropylaniline) [J. Org. Chem. 22, 832 (1957)], 14 g (soit 11,43 cm3) de chlorosulfonate de triméthylsilyle dans 50 cm3 de chlorure de méthylène. Après évaporation à sec, le résidu est repris dans 100 cm3 de chlorure de méthylène, ajoute 14 g de pentachlorure de phosphore et porte 4 heures à ébullition. On évapore à sec, reprend le résidu dans 50 cm3 d'eau, extrait à l'éther, sèche sur sulfate de sodium, filtre et évapore. On obtient 9,6 g de produit attendu que l'on utilise tel quel.

**Exemple 17 : 1-(4-isopropylthiophénylsulfonyl) 2-pyrrolidinone.**

On opère comme à l'exemple 12, en utilisant 1,7 g de 2-pyrrolidinone dans 51 cm3 de tétrahydrofuranne, 13,3 cm3 d'une solution 1,5M de n-butyl lithium dans le n-hexane et 5 g de chlorure de 4-isopropylsulfure benzènesulfonyle dans 5 cm3 de tétrahydrofuranne. On laisse revenir à température ambiante, évapore à sec, reprend le résidu dans l'eau, filtre le précipité et le sèche. On obtient 3,1 g de produit attendu, F = 62¤-66¤C, que l'on recristallise dans l'isopropanol et recueille 2,4 g de produit fondant à 68¤-70¤C.

Analyse : $C_{13}H_{17}NO_3S_2$

| | | | | |
|---|---|---|---|---|
| Calculé | : | C% 52,15 | H% 5,72 | N% 4,68 |
| Trouvé | : | 51,86 | 5,63 | 4,57 |

Le chlorure de 4-isopropyl sulfure benzène sulfonyle utilisé au départ de l'exemple 17 a été préparé comme suit.

On ajoute, goutte à goutte, à une température comprise entre -5¤C et +3¤C, 5,8 g de dioxane à une solution comprenant 5,32 g d'anhydride sulfurique (Coll. Org. Synth. IV, p. 846) dans 24 cm3 de 1,2-dichloroéthane, puis 10 g de sulfure d'isopropylphényle [J. Chem. Soc. (1948), 1820] dissous dans 20 cm3 de 1,2-dichloroéthane sans que la température dépasse +5¤C. On laisse revenir à température ambiante en 1 heure puis chauffe au reflux 1 heure et demie. On évapore à sec, dissout le résidu dans 50 cm3 d'eau, neutralise avec du bicarbonate de soude en solution à 10%, filtre et sèche. On obtient 15 g de sel sodique que l'on traite avec 150 cm3 de chlorure de méthylène et 11,55 g de pentachlorure de phosphore en chauffant 2 heures au reflux. On laisse refroidir à température ambiante, filtre et évapore le solvant. On obtient 5 g de produit pur.

**Exemple 18 : 1-[4-(4-hexahydroazépin) phénylsulfonyl] 2-pyrrolidinone.**

**Stade A** : Acide 4-(4-hexahydroazépinephénylsulfonylamino) butyrique.

On ajoute 6 g de chlorure de 4-hexahydroazépinebenzènesulfonyle à une solution comprenant 2,26 g d'acide 4-aminobutyrique 2,6 g de soude en solution dans 60 cm3 d'eau puis 60 cm3 de tétrahydrofuranne de façon à obtenir une solution. On agite 3 heures à température ambiante, évapore le solvant, acidifie à l'aide d'acide acétique, dilue à l'aide de 100 cm3 d'eau, filtre le précipité et le sèche. On obtient 3,8 g de produit attendu. F = 133¤-135¤C. Après recristallisation dans le mélange éthanol-eau (1-1), F = 134¤-135¤C.

Analyse : $C_{16}H_{24}N_2O_4S$ : 340,454

| | | | | |
|---|---|---|---|---|
| Calculé | : | C% 56,45 | H% 7,10 | N% 8,23 |
| Trouvé | : | 54,55 | 7,12 | 8,22 |

**Stade B** : 1-[4-(4-hexahydroazépin) phénylsulfonyl] 2-pyrrolidinone.

On porte 1 heure au reflux un mélange constitué de 3,5 g de produit obtenu comme au stade A et 3,5 g d'acétate de sodium dans 35 cm3 d'anhydride acétique. On refroidit à température ambiante et évapore à sec. On reprend le résidu dans 30 cm3 d'eau, filtre et sèche. On obtient 3 g de produit que l'on chromatographie sur silice (éluant : acétate d'éthyle). Après cristallisation dans le mélange isopropanol-eau (1-1), on obtient 2 g de produit. F = 155¤-156¤C.

Analyse : $C_{16}H_{22}N_2O_3S$ : 322,438

| | | | | |
|---|---|---|---|---|
| Calculé | : | C% 59,60 | H% 6,88 | N% 8,69 |
| Trouvé | : | 59,66 | 6,94 | 8,66 |

Le chlorure de 4-hexahydroazépinebenzènesulfonyle utilisé au départ de l'exemple 18 a été préparé comme suit :

On ajoute 2,9 g de dioxane dans une solution comprenant 2,64 g d'anhydride sulfurique dans 78 cm3 de chlorure de méthylène refroidie à +5¤C/+10¤C puis ajoute à la même température 5,26 g de 1-phénylhexahydroazépine en solution dans 53 cm3 de chlorure de méthylène. On laisse revenir à température ambiante puis chauffe au reflux pendant 2 heures. On refroidit de nouveau à température ambiante, ajoute 200 cm3 d'éther éthylique, filtre le précipité, le lave à l'éther, le sèche et obtient 7,2 g d'acide fondant à 235¤C (décomp.). On traite cet acide par 36 cm3 d'oxychlorure de phosphore, 36 cm3 de chlorure de méthylène et 5,87 g de pentachlorure de phosphore pendant 4 heures à température ambiante. On évapore à sec, reprend le résidu avec du chloroforme et de l'eau, sépare la phase organique, sèche sur sulfate de sodium, filtre et évapore à sec. On obtient 6,6 g de produit que l'on utilise tel quel. F = 85¤-88¤C.

**Exemple 19 : 1[(4-pipérazino) phénylsulfonyl] 2-pyrrolidinone.**

**Stade A :** Acide 4-[4-(4-benzyloxycarbonylpipérazin-1-yl) phénylsulfonylamino] butyrique.

A une solution comprenant 2,7 g d'acide 4-amino butyrique et 3,18 g de soude dans 50 cm3 d'eau, on ajoute 10,5 g de chlorure 4-[(4-benzyloxycarbonyl) pipérazin-1-yl] benzènesulfonyle puis 70 cm3 de tétrahydrofuranne afin d'obtenir une solution. On agite 1 heure à température ambiante, évapore le solvant, acidifie à l'aide d'acide acétique, extrait au chloroforme, sèche la phase organique sur sulfate de sodium, filtre et évapore à sec. On obtient 8,2 g de produit attendu.

**Stade B :** 1-[(4-pipérazino) phénylsulfonyl] 2-pyrrolidinone.

On chauffe 30 minutes au reflux 8,2 g de produit obtenu au stade A, 8,2 g d'acétate de sodium et 123 cm3 d'anhydride acétique. On refroidit à température ambiante, évapore à sec, reprend le résidu avec 100 cm3 d'eau, filtre et sèche. On obtient 5,6 g de 1-[4-(4-benzyloxycarbonylpipérazin-1-yl) phénylsulfonyl] 2-pyrrolidinone. F = 153¤-155¤C. Après recristallisation dans le mélange éthanol-acétone (10-1), on obtient 3,7 g de produit fondant à 158¤-160¤C. On met en suspension 7 g de produit préparé comme ci-dessus et 1 g de palladium dans 105 cm3 de diméthylacétamide, ajoute quelques gouttes de triéthylamine, puis hydrogène sous pression ambiante (à raison de 280 cm3 d'hydrogène). On filtre le catalyseur, évapore à sec et obtient 2 g de produit que l'on dissout dans l'acide acétique, évapore à sec, reprend le résidu dans l'alcool isopropylique et obtient 1,5 g de produit cristallisé. F = 108¤C.

Analyse : $C_{14}H_{19}N_3O_3S$, $CH_3COOH$, 0,5 $H_2O$ : 378,46

| | | | | |
|---|---|---|---|---|
| Calculé | : | C% 50,78 | H% 6,39 | N% 11,10 |
| Trouvé | : | 50,39 | 6,15 | 11,14 |

Le chlorure de 4-[(4-benzyloxycarbonyl) pipérazin-1-yl] benzènesulfonyle utilisé au départ de l'exemple 19 a été préparé comme suit :

Stade a : 4-carbobenzyloxy 1-phénylpipérazine.

Dans une solution comprenant 4,05 g de N-phénylpipérazine dans 50 cm3 de benzène et 2,5 g de triéthylamine, on ajoute une solution de 4,4 g de chloroformiate de benzyle dans le toluène à une température comprise entre +5¤C et +10¤C. On laisse revenir à température ambiante, agite pendant 2 heures, filtre le chlorure de triéthylamine et évapore à sec. On obtient un résidu huileux que l'on concrète dans le n-hexane et récupère 7,1 g de produit attendu. F = 49¤-51¤C. Après recristallisation dans le mélange éther éthylique-n-hexane, F = 51¤-52¤C.

Analyse : $C_{18}H_{20}N_2O_2$ : 296,37

| | | | | |
|---|---|---|---|---|
| Calculé | : | C% 72,95 | H% 6,80 | N% 9,45 |
| Trouvé | : | 73,13 | 6,79 | 9,38 |

Stade b : Chlorure de 4-[(4-benzyloxycarbonyl) pipérazin-1-yl] benzènesulfonyle.

On ajoute une solution comprenant 3,04 g d'anhydride sulfurique dans 90 cm3 de chlorure de méthylène à 11 g de produit obtenu au stade a) en solution dans 110 cm3 de chlorure de méthylène puis 3,6 g de dioxane à une température comprise entre +5¤C et +10¤C. On laisse revenir à température ambiante puis chauffe au reflux pendant 2 heures. On refroidit à température ambiante, dilue par 300 cm3 d'éther éthylique, filtre et sèche. On obtient 13,7 g de produit fondant à 210¤C que l'on traite par 100 cm3 d'oxychlorure de phosphore et 7,57 g de pentachlorure de phosphore pendant 4 heures à température ambiante. On évapore à sec, reprend le résidu aved du chloroforme et de l'eau, sépare la phase organique, sèche sur sulfate de sodium, filtre et évapore à sec. On obtient 10,6 g de produit que l'on utilise tel quel.

**Exemples de compositions pharmaceutiques.**

a) On a préparé des comprimés répondant à la formule suivante :

| | |
|---|---|
| - Produit de l'exemple 1 ..... | 10 mg |
| - Excipient q.s. pour un comprimé terminé à ..... | 300 mg |

(Détail de l'excipient : lactose, amidon de blé, amidon traité, amidon de riz, stéarate de magnésium, talc).

b) On a préparé des gélules répondant à la formule suivante :

| | |
|---|---|
| - Produit de l'exemple 1 ..... | 20 mg |
| - Excipient q.s. pour une gélule terminée à ..... | 300 mg |

(Détail de l'excipient : talc, stéarate de magnésium, aérosil).

## ETUDES BIOCHIMIQUES ET PHARMACOLOGIQUES

1) Liaison à des différents récepteurs cérébraux

Nous reportons dans le tableau suivant les résultats obtenus par deux exemples significatifs de l'invention.

a) Récepteur muscarinique 1

Sa préparation est faite à partir de Cortex prélevés sur des cerveaux de Rat mâle pesant 150 à 200 g, (Iffa Crédo) broyé au Polytron dans un tampon Na/K 10 mM pH 7,4. Après incubation (aliquotes de 0,5 ml d'homogénat) pendant 60 minutes à 25¤C en présence de 0,25 nM de $^3H$ pirenzépine soit seule, soit avec le produit à tester, soit avec un excès de pirenzépine à $10^{-5}M$ (pour déterminer la radioactivité fixée non spécifique), les incubats sont refroidis et filtrés.

La filtration est effectuée sur filtres Whatman GF/C prélavés dans une solution de polyéthylène imine à 0,05 %. Les filtres sont rincés par 3 x 5 ml de tampon phosphate Na/k 10 mM pH 7,4 puis on effectue les mesures par scintillation liquide.

b) Récepteur muscarinique 2

La préparation est effectuée à partir de cerveaux de Rat mâle pesant 150 à 200 g (Iffa Crédo).

Les cerveaux sont broyés (Téflon-verre) dans une solution de sucrose 0,32 M. L'homogénat est centrifugé 10 minutes à 1000 g (0 - 4¤C).

Le surnageant obtenu est recueilli et centrifugé à 30 000 g pendant 15 minutes (0 - 4¤C).

Le culot est remis en suspension dans un tampon Tris 50 mM pH 7,5 et le nouvel homogénat est centrifugé de nouveau à 30 000 g pendant 15 minutes (0 - 4¤C).

Les culots, après élimination du surnageant, peuvent être utilisés aussitôt ou conservés jusqu'à 1 mois à -30¤C.

Pour une expérience les culots sont d'abord décongelés, si nécessaire, à température ambiante et remis en suspension à l'aide d'un Dounce dans du tampon Tris 50 mM pH 7,5. Des aliquotes de 2 ml sont mis à incuber 60 minutes à 25¤C en présence de 0,3 nM de $^3H$ quinuclidinyl benzylate soit seul, soit avec le produit à tester, soit avec de la benzatropine à $10^{-5}M$ pour déterminer la radioactivité fixée non spécifique.

A la fin de l'incubation les tubes d'incubats sont refroidis à 4¤C et filtrés rapidement sur filtres Whatman GF/C. Les filtres sont rincés par 3 x 5 ml de tampon Tris 50 mM pH 7,5 puis on effectue les mesures par scintillation liquide (Henry I Yamamura, Solomon H. Snyder, Proc. Nat. Acad. Sc. (1974) 71 n¤ 5, 1725 - 1729).

Les résultats sont exprimés en $CI_{50}$ (concentration nécessaire pour inhiber de 50 % la radioactivité spécifique fixée).

TABLEAU 1

| Composé de l'exemple | Affinité pour les récepteurs muscariniques $M_1$ et $M_2$ | |
|---|---|---|
| | /$^3$H/Pirenze-pine | /$^3$H/.Quinucli-dinyl benzylate |
| 1 | 130 | 1300 |
| 2 | 98 | 1300 |

Les composés des exemples 1 et 2 montrent une remarquable et intéressante affinité pour le récepteur muscarinique, et principalement pour le récepteur de type $M_1$. Par contre les mêmes composés ont montré une affinité négligeable ($CI_{50}$ 5000-10000) pour les autres récepteurs examinés parmi lesquels on peut citer ceux de la dopamine, de la sérotonine (5 $HT_1$ et 5 $HT_2$), des benzodiazépines, du GABA, les adrénorécepteurs (alpha 1, alpha 2, béta 1, béta 2) ou encore les récepteurs opiacés (mu, kappa).

2) Intéraction et affinité avec différents récepteurs intestinaux

L'intéraction des composés avec différents récepteurs a été évaluée sur l'iléon isolé de cobaye selon la méthode suivante.

Des segments d'iléon de cobayes de 2,5-3 cm, ont été lavés et immédiatement suspendus dans un bain contenant 10 ml d'une solution de Tyrode à 37¤C et aérée avec un mélange d'oxygène (95 %) et de gaz carbonique (5 %). Après une période de stabilisation de 30 minutes au moins ou enregistre les contractions, en maintenant la préparation sous tension constante de 1 g, à l'aide d'un capteur relié à un polygraphe. On a évalué l'action agoniste en laissant le composé en contact avec le tissu isolé pendant une période nécessaire à exprimer la concentration maximale ; ensuite, on a lavé avec la solution de Tyrode. On n'a ajouté la dose suivante au bain qu'après que la préparation fût revenu sur la ligne de base. Comme produit de référence on a employé l'arécoline. On a évalué l'action antagoniste sur les contractions induites par l'acétylcholine ($1x10^{-6}$M), l'histamine ($1x10^{-5}$M la sérotonine ($1x10^{-6}$M) et le chlorure de baryum ($2x10^{-4}$M). L'atropine, le diphénydramine, le méthysergide et la papavérine ont été employés comme produits de référence. Le temps de contact avant d'ajouter l'agoniste a été d'une minute.

Pour chaque composé les courbes dose-réponse sont obtenues avec 4 à 6 concentrations différentes et 3 à 5 épreuves indépendantes. L'activité agoniste est exprimée par le $pD_2$ (logarithme négatif de la dose qui produit 50 % de l'effet maximum). L'activité antagoniste est exprimée par la $CI_{50}$ (concentration inhibant 50 % de la réponse maximale). Les résultats obtenus avec les composés des exemples 1 et 2 sont reportés dans le tableau suivant :

TABLEAU 2

| Composé de l'exemple | Antagonisme à différents agents ($CI_{50}$:M) | | | | Action agoniste ($pD_2$) |
|---|---|---|---|---|---|
| | Ach | Istam. | Seroton. | $BaCl_2$ | |
| 1 | $6,4x10^{-8}$ | $> 10^{-4}$ | $> 10^{-4}$ | $1,6x10^{-5}$ | $< 4$ |
| 2 | $6,2x10^{-8}$ | $> 10^{-4}$ | $> 10^{-4}$ | $6,0x10^{-6}$ | $< 4$ |
| Atropine | $9,5x10^{-9}$ | | | | |
| Diphénydra-mine | | $8,3x10^{-7}$ | | | |
| Métisergide | | | $1,1x10^{-7}$ | | |
| Papaverine | | | | $4,5x10^{-5}$ | |
| Arécoline | | | | | 6,68 |

Les études "in vitro" sur l'iléon isolé de cobaye ont mis en évidence que les composés de l'invention sont des puissants agents antimuscariniques. Ils antagonisent les contractions induites par l'acétylcholine mais non celles induites par l'histamine, la sérotonine. Ces composés ont montré une activité antagoniste un peu inférieure ($\simeq$ 7 fois) à celle de l'atropine.

La remarquable action antagoniste des composés de l'invention a été confirmée sur le colon isolé de rat. Dans ce test nous avons établi que l'activité antagoniste est de type compétitif ou non compétitif.

Nous avons employé la méthode suivante : des segments de colon de rat de 2,5 cm environ sont lavés et suspendus dans un bain isolés contenant 10 ml d'une solution de De Jalon ayant la composition suivante : (mM) : NaCl 154 ; KCl 5,7 ; $CaCl_2$ 0,27 ; $NaHCO_3$ 5,9 et glucose 2,5. La température du bain est maintenue à 32¤C. Dans ces conditions, en maintenant le préparé sous une tension de 1 g, l'activité spontanée du colon est minimale. Après une période de stabilisation de 30 min au moins, on enregistre les variations de tension à

l'aide d'un transducteur isométrique relié à un enregistreur. On a effectué une série d'essais pour évaluer l'activité antagoniste par rapport aux contractions induites par une dose maximale d'acétylcholine. ($3x10^{-6}M$).

On laisse les composés en contact avec le préparé pour une période de 3 minutes avant d'ajouter l'acétylcholine.

Comme produits de référence nous avons employé l'atropine. Pour chaque composé les courbes dose-réponse sont obtenues avec 4 à 6 concentrations différentes et 3 à 5 essais indépendants.

L'activité antagoniste est exprimée comme $CI_{50}$ (concentration inhibant de 50 % la réponse maximale induite par l'acétylcholine).

Dans une seconde série d'essais l'acétylcholine est ajoutée au bain en doses cummulatives selon la méthode de van Rossum (J.M. van Rossum, Arch. Int. Pharmacodyn. : 143, 299, 1963). Après avoir obtenu 2 courbes dose-réponse égales et consécutives par l'acétylkoline une troisième courbe dose-réponse est obtenue en présence du composé (temps de contact du composé avant l'acétylkoline = 5 min). Chaque composé a été essayé avec 3-4 concentrations différentes.

L'affinité antagoniste et le type d'antagonisme (compétitif ; non compétitif) pour les récepteurs muscariniques a été calculée selon la méthode de Schild (H.D. Schild, Brit.J. Pharmacol. 2, 189, 1947).

TABLEAU 3

| Composé de l'exemple | Colon isolé de rat | | |
|---|---|---|---|
| | $CI_{50}$ | $pA_2$ | "Slope" |
| 1 | $2,8x10^{-7}$ | 7,77 | 0,89 |
| 2 | $1,6x10^{-7}$ | 7,35 | 1,08 |
| Atropine | $2,7x10^{-8}$ | 8,31 | 0,97 |

Les deux composés de l'exemple 1 et 2, ainsi comme l'atropine, ont donné un déplacement paralèle vers la droite de la courbe dose-réponse de l'acétylcholine sans réduire la contraction maximale.

La pente des droites de régression dans les "Schild plot" correspond à la valeur théorique de 1 (tab. 3). D'après ces données et les valeurs de $PA_2$ obtenus (tab. 3) nous pouvons conclure que les composés de l'invention sont des antagonistes compétitifs pour les récepteurs muscariniques, impliqués dans les contractions du colon de rat induites par l'acétylcholine, et ils montrent une puissance d'environ 4 à 10 fois inférieure à celle de l'atropine.

3) Action anticholinergique "in vivo".

L'activité anticholinergique des composés à été déterminée en évaluant la capacité d'inhiber les effets cholinomimétiques par le carbachol. Le sulfate d'atropine a été employé comme produit de référence.

On utilise ses souris mâles $CD_1$ pesant 25 à 30 g. Elles sont réparties en groupes de 5 animaux et traitées par voie intrapéritonéale à des doses scalaires des produits ou 0,25% de Méthocel pour les témoins. On utilise 10 animaux pour chaque dose. 30 minutes après l'administration des composés, on injecte aux souris par voie sous-cutanée 1 mg/kg de carbachol, dissous dans du sérum physiologique.

Chaque animal a été examiné 30 minutes après l'injection de carbachol pour évaluer la présence de diarrhée, salivation et larmoiement ; on a mesuré aussi la température corporelle au moyen d'un thermo couple inséré de 1,5 cm dans le rectum.

Le carbachol (1mg/kg s.c.) a induit diarrhée, salivation et larmoiement chez toutes les souris témoins et une diminution de la température rectale de 2,5¤C environ.

Pour chaque composé, nous avons déterminé et reporté dans le tableau suivant la dose capable d'inhiber chez 50% des animaux, l'apparition des symptomes cholinomimétiques périphériques induits par le carbachol et d'augmenter de 1¤C l'effet hypothermique induit par l'agent cholinergique.

TABLEAU 4

| Composé de l'exemple | Dose mg/kg i.p. | | | Température corporelle |
|---|---|---|---|---|
| | Diarrhée | salivation | larmoiement | |
| 1 | 7 | 10 | > 50 | 50 |
| 2 | 7 | > 50 | > 50 | > 50 |
| 10 | 3 | 50 | > 50 | > 50 |
| 18 | 1 | 15 | 50 | 15 |
| Atropine | 0,04 | 0,06 | 0,05 | 0,03 |

Les résultats obtenus montrent que, contrairement à l'atropine, les composés des exemples en particulier ceux des exemples 1,2 et 10 exercent "in vivo" une action anticholinergique sélective au niveau de la musculature intestinale.

## Revendications

1.- Les composés de formule (I) :

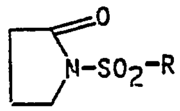

(I)

dans laquelle R représente
- ou bien un radical

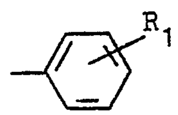

dans laquel $R_1$ en position quelconque sur le noyau phényle représente
soit un radical alcoyle renfermant jusqu'à 8 atomes de carbone linéaire, ramifié ou cyclique, saturé ou insaturé,
soit un radical

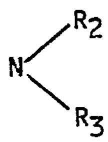

dans lequel $R_2$ et $R_3$ identiques ou différents représentent un atome d'hydrogène, un radical alcoyle linéaire, saturé ou insaturé renfermant jusqu'à 8 atomes de carbone, ou forment ensemble avec l'atome d'azote auquel ils sont liés un radical hétérocyclique carboné renfermant éventuellement un autre hétéroatome,
soit un radical $NO_2$
soit un radical OR', R' représentant un atome d'hydrogène, un radical alcoyle linéaire ramifié ou cyclique renfermant jusqu'à 8 atomes de carbone, ou un radical aryle renfermant jusqu'à 14 atomes de carbone,
soit un radical $SR_4$ ou $S(O)R_5$, $R_4$ et $R_5$ représentant un radical alcoyle linéaire, ramifié ou cyclique, saturé ou insaturé renfermant jusqu'à 8 atomes de carbone,
- ou bien R représente un radical naphtyle, éventuellement substitué par un radical $R'_1$, $R'_1$ pouvant prendre l'une des valeurs indiquées ci-dessus pour $R_1$.

2.- Les composés de formule (I) tels que définis à la revendication 1, dans lesquels R représente un radical

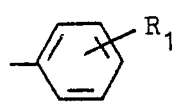

$R_1$ conservant la même signification que dans la revendication 1.

3.- Les composés de formule (I) tels que définis à la revendication 2, dans lesquels le radical $R_1$ est en position 4.

4.- Les composés de formule (I) tels que définis à la revendication 2 ou 3, dans lesquels $R_1$ représente un radical alcoyle linéaire ou ramifié renfermant jusqu'à 8 atomes de carbone et notamment un radical tert-butyle.

5.- Les composés de formule (I) tels que définis à la revendication 2 ou 3, dans lesquels $R_1$ représente un radical

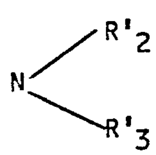

dans lesquels R'₂ et R'₃ représentent un radical alcoyle linéaire ou ramifié renfermant jusqu'à 8 atomes de carbone ou forment avec l'atome d'azote auquel ils sont liés un radical hétérocyclique.

6.- Les composés de formule (I) tels que définis à la revendication 2 ou 3, dans lesquels $R_1$ représente un radical $SR_4$, $R_4$ représentant un radical alcoyle linéaire ou ramifié renfermant jusqu'à 4 atomes de carbone et notamment un radical méthyle.

7.- Les composés de formule (I) tels que définis à la revendication 1, dont les noms suivent :
- la 1-(4-tertbutylbenzènesulfonyl) 2-pyrrolidinone,
- la 1-[4-(diéthylamino) benzènesulfonyl] 2-pyrrolidinone,
- la 1-[4-(diméthylamino) benzènesulfonyl] 2-pyrrolidinone,
- la 1-[4-(méthylthio) benzènesulfonyl] 2-pyrrolidinone,
- la 1-[4-(1-pipéridinyl) phénylsulfonyl] 2-pyrrolidinone.

8.- Procédé de préparation des composés de formule (I), tels que définis à la revendication 1, caractérisé en ce que l'on soumet la 2-pyrrolidinone

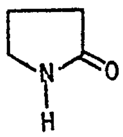

à l'action d'un composé de formule (II) :
$R\text{-}SO_2Hal$ (II)
dans laquelle Hal représente un atome de chlore ou de brome, et R conserve la même signification que précédemment, pour obtenir le composé de formule (I) correspondant.

9.- Procédé de préparation des composés de formule (I) tels que définis à la revendication 1, caractérisé en ce que l'on soumet l'acide 4-aminobutyrique à l'action d'un composé de formule (II) :
$R\text{-}SO_2\text{-}Hal$ (II)
dans laquelle R et Hal ont la signification indiquée dans la revendication 1, pour obtenir un composé de formule (III) :

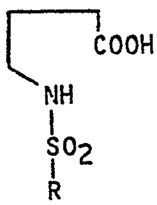

(III)

dans laquelle R a la signification indiquée dans la revendication 1, que l'on cyclise pour obtenir le produit de formule (I) correspondant.

10.- A titre de médicaments, les composés de formule (I) tels que définis à l'une quelconque des revendications 1 à 6.

11.- A titre de médicaments, les composés de formule (I) tels que définis à la revendication 7.

12.- Les compositions pharmaceutiques renfermant au moins un médicament tel que défini à la revendication 10 ou 11.

13.- A titre de produits industriels nouveaux, les produits de formule (III) tels que définis à la revendication 9.

**Revendications pour l'Etat contractant suivant: ES**

1.- Procédé de préparation des composés de formule (I) :

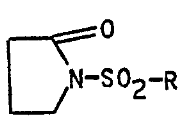

(I)

dans laquelle R représente
- ou bien un radical

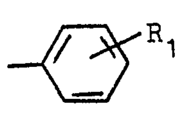

dans laquel $R_1$ en position quelconque sur le noyau phényle représente
soit un radical alcoyle renfermant jusqu'à 8 atomes de carbone linéaire, ramifié ou cyclique, saturé ou

insaturé,
soit un radical

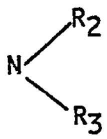

dans lequel $R_2$ et $R_3$ identiques ou différents représentent un atome d'hydrogène, un radical alcoyle linéaire, saturé ou insaturé renfermant jusqu'à 8 atomes de carbone, ou forment ensemble avec l'atome d'azote auquel ils sont liés un radical hétérocyclique carboné renfermant éventuellement un autre hétéroatome,
soit un radical $NO_2$,
soit un radical OR', R' représentant un atome d'hydrogène, un radical alcoyle linéaire ramifié ou cyclique renfermant jusqu'à 8 atomes de carbone, ou un radical aryle renfermant jusqu'à 14 atomes de carbone,
soit un radical $SR_4$ ou $S(O)R_5$, $R_4$ et $R_5$ représentant un radical alcoyle linéaire, ramifié ou cyclique, saturé ou insaturé renfermant jusqu'à 8 atomes de carbone,
- ou bien R représente un radical naphtyle, éventuellement substitué par un radical $R'_1$, $R'_1$ pouvant prendre l'une des valeurs indiquées ci-dessus pour $R_1$, caractérisé en ce que l'on soumet la 2-pyrrolidinone

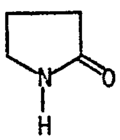

à l'action d'un composé de formule (II) :
dans laquelle Hal représente un atome de chlore ou de brome, et R conserve la même signification que précédemment, pour obtenir le composé de formule (I) correspondant ou bien l'on soumet l'acide 4-amino-butyrique à l'action d'un composé de formule (II) :
$R-SO_2-Hal$ (II)
dans laquelle R et Hal ont la signification indiquée dans la revendication 1, pour obtenir un composé de formule (III) :

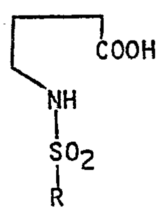

(III)

dans laquelle R a la signification indiquée dans la revendication 1, que l'on cyclise pour obtenir le produit de formule (I) correspondant.

2.- Procédé selon la revendication 1, pour la préparation des produits de formule (I) telle que définie à la revendication 1, caractérisé en ce que l'on soumet la 2-pyrrolidinone

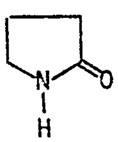

à l'action d'un composé de formule (II) :
$R-SO_2Hal$ (II)
dans laquelle Hal représente un atome de chlore ou de brome, et R conserve la même signification que précédemment, pour obtenir le composé de formule (I) correspondant.

3.- Procédé selon la revendication 1, pour la préparation des produits de formule (I) telle que définie à la revendication 1, caractérisé en ce que l'on soumet l'acide 4-amino-butyrique à l'action d'un composé de formule (II) :
$R-SO_2-Hal$ (II)
dans laquelle R et Hal ont la signification indiquée dans la revendication 1, pour obtenir un composé de formule (III) :

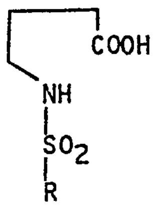

(III)

dans laquelle R a la signification indiquée dans la revendication 1, que l'on cyclise pour obtenir le produit de formule (I) correspondant.

4.- Procédé selon la revendication 2, caractérisé en ce que l'on utilise au départ un composé de formule (II) dans laquelle R représente un radical

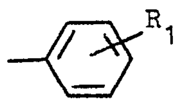

$R_1$ conservant la même signification que dans la revendication 1.

5.- Procédé selon la revendication 3, caractérisé en ce que l'on utilise au départ un composé de formule (II) dans laquelle R représente un radical

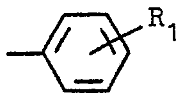

$R_1$ conservant la même signification que dans la revendication 1.

6.- Procédé selon la revendication 4, caractérisé en ce que le radical $R_1$ est en position 4.

7.- Procédé selon la revendication 5, caractérisé en ce que le radical $R_1$ est en position 4.

8.- Procédé selon la revendication 4 ou 6, caractérisé en ce que $R_1$ représente un radical alcoyle linéaire ou ramifié renfermant jusqu'à 8 atomes de carbone et notamment un radical tert-butyle.

9.- Procédé selon la revendication 5 ou 7, caractérisé en ce que $R_1$ représente un radical alcoyle linéaire ou ramifié renfermant jusqu'à 8 atomes de carbone et notamment un radical tert-butyle.

10.- Procédé selon la revendiction 4 ou 6, caractérisé en ce que $R_1$ représente un radical

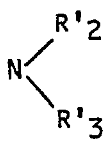

dans lesquels $R'_2$ et $R'_3$ représentant un radical alcoyle linéaire ou ramifié renfermant jusqu'à 8 atomes de carbone ou forment avec l'atome d'azote auquel ils sont liés un radical hétérocyclique.

11.- Procédé selon la revendication 5 ou 7, caractérisé en ce que $R_1$ représente un radical

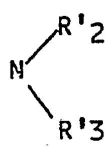

dans lesquels $R'_2$ et $R'_3$ représentent un radical alcoyle linéaire ou ramifié renfermant jusqu'à 8 atomes de carbone ou forment avec l'atome d'azote auquel ils sont liés un radical hétérocyclique.

12.- Procédé selon la revendication 4 ou 6, caractérisé en ce que $R_1$ représente un radical $SR_4$, $R_4$ représentant un radical alcoyle linéaire ou ramifié renfermant jusqu'à 4 atomes de carbone et notamment un radical méthyle.

13.- Procédé selon la revendication 5 ou 7, caractérisé en ce que $R_1$ représente un radical $SR_4$, $R_4$ représentant un radical alcoyle linéaire ou ramifié renfermant jusqu'à 4 atomes de carbone et notamment un radical méthyle.

14.- Procédé selon la revendication 2, caractérisé en ce que l'on choisit le produit de formule (II) de manière telle que l'on prépare les composés de formule (I) tels que définis à la revendication 1, dont les noms suivent :

- la 1-(4-tertbutylbenzènesulfonyl) 2-pyrrolidinone,
- la 1-[4-diéthylamino) benzènesulfonyl] 2-pyrrolidinone,
- la 1-[4-diméthylamino) benzènesulfonyl] 2-pyrrolidinone,
- la 1-[4-méthylthio) benzènesulfonyl] 2-pyrrolidinone,

- la 1-[4-(1-pipéridinyl) phénylsulfonyl] 2-pyrrolidinone.

15.- Procédé selon la revendication 3, caractérisé en ce que l'on choisit le produit de formule (II) de manière telle que l'on prépare les composés de formule (I) tels que définis à la revendication 1, dont les noms suivent :

- la 1-(4-tertbutylbenzènesulfonyl] 2-pyrrolidinone,
- la 1-[4-diéthylamino) benzènesulfonyl] 2-pyrrolidinone,
- la 1-[4-diméthylamino) benzènesulfonyl] 2-pyrrolidinone,
- la 1-[4-méthylthio) benzènesulfonyl] 2-pyrrolidinone,
- la 1-[4-(1-pipéridinyl) phénylsulfonyl] 2-pyrrolidinone.

**Revendications pour l'Etat contractant suivant: GR**

1.- Procédé de préparation des composés de formule (I) :

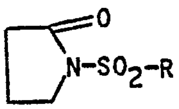

(I)

dans laquelle R représente

- ou bien un radical

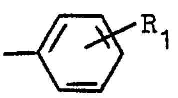

dans laquel $R_1$ en position quelconque sur le noyau phényle représente
soit un radical alcoyle renfermant jusqu'à 8 atomes de carbone linéaire, ramifié ou cyclique, saturé ou insaturé,
soit un radical

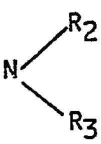

dans lequel $R_2$ et $R_3$ identiques ou différents représentent un atome d'hydrogène, un radical alcoyle linéaire, saturé ou insaturé renfermant jusqu'à 8 atomes de carbone, ou forment ensemble avec l'atome d'azote auquel ils sont liés un radical hétérocyclique carboné renfermant éventuellement un autre hétéroatome,
soit un radical $NO_2$,
soit un radical OR', R' représentant un atome d'hydrogène, un radical alcoyle linéaire ramifié ou cyclique renfermant jusqu'à 8 atomes de carbone, ou un radical aryle renfermant jusqu'à 14 atomes de carbone,
soit un radical $SR_4$ ou $S(O)R_5$, $R_4$ et $R_5$ représentant un radical alcoyle linéaire, ramifié ou cyclique, saturé ou insaturé renfermant jusqu'à 8 atomes de carbone,

- ou bien R représente un radical naphtyle, éventuellement substituée par un radical $R'_1$, $R'_1$ pouvant prendre l'une des valeurs indiquées ci-dessus pour $R_1$, caractérisé en ce que l'on soumet la 2-pyrrolidinone

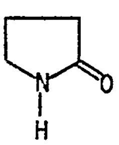

à l'action d'un composé de formule (II) :
dans laquelle Hal représente un atome de chlore ou de brome, et R conserve la même signification que précédemment, pour obtenir le composé de formule (I) correspondant ou bien l'on soumet l'acide 4-amino-butyrique à l'action d'un composé de formule (II) :
$R-2O_2-Hal$ (II)
dans laquelle R et Hal ont la signification indiquée dans la revendication 1, pour obtenir un composé de formule (III) :

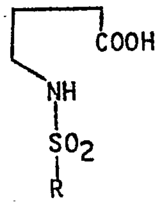

(III)

dans laquelle R a la signification indiquée dans la revendication 1, que l'on cyclise pour obtenir le produit de formule (I) correspondant.

2.- Procédé selon la revendication 1, pour la préparation des produits de formule (I) telle que définie à la revendication 1, caractérisé en ce que l'on soumet la 2-pyrrolidinone

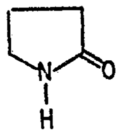

à l'action d'un composé de formule (II) :

$R-SO_2Hal$ (II)

dans laquelle Hal représente un atome de chlore ou de brome, et R conserve la même signification que précédemment, pour obtenir le composé de formule (I) correspondant.

3.- Procédé selon la revendication 1, pour la préparation des produits de formule (I) telle que défini à la revendication 1, caractérisé en ce que l'on soumet l'acide 4-amino-butyrique à l'action d'un composé de formule (II) :

$R-SO_2-Hal$ (II)

dans laquelle R et Hal ont la signification indiquée dans la revendication 1, pour obtenir un composé de formule (III) :

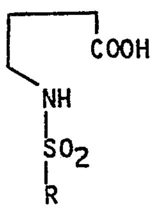

(III)

dans laquelle R a la signification indiquée dans la revendication 1, que l'on cyclise pour obtenir le produit de formule (I) correspondant.

4.- Procédé selon la revendication 2, caractérisé en ce que l'on utilise au départ un composé de formule (II) dans laquelle R représente un radical

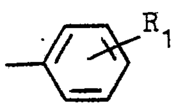

$R_1$ conservant la même signification que dans la revendication 1.

5.- Procédé selon la revendication 3, caractérisé en ce que l'on utilise au départ un composé de formule (II) dans laquelle R représente un radical

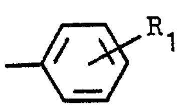

$R_1$ conservant la même signification que dans la revendication 1.

6.- Procédé selon la revendication 4, caractérise en ce que le radical $R_1$ est en position 4.

7.- Procédé selon la revendication 5, caractérisé en ce que le radical $R_1$ est en position 4.

8.- Procédé selon la revendication 4 ou 6, caractérisé en ce que $R_1$ représente un radical alcoyle linéaire ou ramifié renfermant jusqu'à 8 atomes de carbone et notamment un radical tert-butyle.

9. Procédé selon la revendication 5 ou 7, caractérisé en ce que $R_1$ représente un radical alcoyle linéaire ou ramifié renfermant jusqu'à 8 atomes de carbone et notamment un radical tert-butyle.

10.- Procédé selon la revendication 4 ou 6, caractérisé en ce que $R_1$ représente un radical

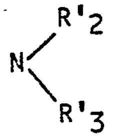

dans lesquels $R'_2$ et $R'_3$ représentent un radical alcoyle linéaire ou ramifié renfermant jusqu'à 8 atomes de carbone ou forment avec l'atome d'azote auquel ils sont liés un radical hétérocyclique.

11.- Procédé selon la revendication 5 ou 7, caractérisé en ce que $R_1$ représente un radical

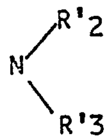

dans lesquels $R'_2$ et $R'_3$ représentent un radical alcoyle linéaire ou ramifié renfermant jusqu'à 8 atomes de carbone ou forment avec l'atome d'azote auquel ils sont liés un radical hétérocyclique.

12.- Procédé selon la revendication 4 ou 6, caractérisé en ce que $R_1$ représente un radical $SR_4$, $R_4$ représentant un radical alcoyle linéaire ou ramifié renfermant jusqu'à 4 atomes de carbone et notamment un radical méthyle.

13.- Procédé selon la revendication 5 ou 7, caractérisé en ce que $R_1$ représente un radical $SR_4$, $R_4$ représentant un radical alcoyle linéaire ou ramifié renfermant jusqu'à 4 atomes de carbone et notamment un radical méthyle.

14.- Procédé selon la revendication 2, caractérisé en ce que l'on choisit le produit de formule (II) de manière telle que l'on prépare les composés de formule (I) tels que définis à la revendication 1, dont les noms suivent :

- la 1-(4-tertbutylbenzènesulfonyl] 2-pyrrolidinone,
- la 1-[4-(diéthylamino) benzènesulfonyl] 2-pyrrolidinone,
- la 1-[4-(diméthylamino) benzènesulfonyl] 2-pyrrolidinone,
- la 1-[4-(méthylthio) benzènesulfonyl] 2-pyrrolidinone,
- la 1-[4-(1-pipéridinyl) phénylsulfonyl] 2-pyrrolidinone.

15.- Procédé selon la revendication 3, caractérisé en ce que l'on choisit le produit de formule (II) de manière telle que l'on prépare les composés de formule (I) tels que définis à la revendication 1, dont les noms suivent :

- la 1-(4-tertbutylbenzènesulfonyl] 2-pyrrolidinone,
- la 1-[4-(diéthylamino) benzènesulfonyl] 2-pyrrolidinone,
- la 1-[4-(diméthylamino) benzènesulfonyl] 2-pyrrolidinone,
- la 1-[4-(méthylthio) benzènesulfonyl] 2-pyrrolidinone,
- la 1-[4-(1-pipéridinyl) benzènesulfonyl] 2-pyrrolidinone.

16.- Procédé de préparation des compositions pharmaceutiques, caractérisé en ce que l'on met à titre de principe actif, l'un au moins des composés de formule (I) telle que définie à la revendication 1, sous une forme destinée à cet usage.

17.- Procédé selon la revendication 16, caractérisé en ce que l'on utilise à titre de principe actif l'un au moins des dérivés de formule (I) tels qu'obtenus à la revendication 14.

18.- Procédé selon la revendication 16, caractérisé en ce que l'on utilise à titre de principe actif l'un au moins des dérivés de formule (I) tels qu'obtenus à la revendication 15.

19.- A titre de produits industriels nouveaux, les produits de formule (III) tels que définis à la revendication 9.